(19) **Europäisches Patentamt European Patent Office Office européen des brevets**

(11) **EP 4 686 719 A1**

(12) **EUROPEAN PATENT APPLICATION**

(43) Date of publication:
04.02.2026 Bulletin 2026/06

(21) Application number: 24382863.9

(22) Date of filing: 02.08.2024

(51) International Patent Classification (IPC):
*C07C 273/04* $^{(2006.01)}$    *B01J 35/23* $^{(2024.01)}$

(52) Cooperative Patent Classification (CPC):
**C07C 273/04; B01J 35/23**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Universitat Politècnica de Catalunya**
08034 Barcelona (ES)
• **B. Braun Surgical, S.A.U.**
08191 Rubí (Barcelona) (ES)

(72) Inventors:
• **ARNAU, Marc**
08034 Barcelona (Barcelona) (ES)
• **SANS MILÀ, Jordi**
08034 Barcelona (Barcelona) (ES)
• **ALEMÁN LLANSÓ, Carlos**
08034 Barcelona (Barcelona) (ES)
• **TURÓN DOLS, Pau**
08191 Rubí (Barcelona) (ES)

(74) Representative: **TRBL Intellectual Property**
**Plaza de Castilla 3, 7°A**
**28046 Madrid (ES)**

(54) **SYNTHESIS OF UREA FROM CARBON DIOXIDE (CO2) AND AMMONIA (NH3) IN THE PRESENCE OF A PERMANENTLY POLARISED CATALYST**

(57)    The present invention refers to a method for the synthesis of urea from carbon dioxide ($CO_2$) and ammonia ($NH_3$), wherein the method comprises contacting a gaseous stream of $CO_2$ with an aqueous solution of $NH_3$ in the presence of a permanently polarised catalyst at a temperature of 70-220°C and at a total gas pressure of 0.01-250 bar. In a further aspect, the invention refers to the use of a permanently polarised catalyst for: reducing $CO_2$ present in a gaseous stream; reducing $NH_3$ present in an aqueous solution; and/or producing urea, by implementing a method according to the invention.

FIG. 9

**Description**

**FIELD OF THE INVENTION**

The present invention belongs to the field of catalysed chemical reactions for the revalorisation of $CO_2$. More specifically, the invention relates to the technical field of catalysed chemical reactions for the production of urea from carbon dioxide ($CO_2$) and ammonia ($NH_3$).

**BACKGROUND OF THE INVENTION**

The conversion of carbon dioxide ($CO_2$) into value-added chemical products, also known as COz-revalorisation, is presented as an attractive and necessary strategy for climate change mitigation. COz-revalorisation not only promotes the removal of $CO_2$ but also favours the reintroduction of $CO_2$ into the economy as a valuable raw material, by reusing it in several sustainable carbon cycles intended to contribute to the strategy zero-impact emissions necessary to maintain the quality standards and energy demands of the 21st century society. Nonetheless, the activation of the thermodynamically stable $CO_2$ molecule requires energy-intensive conditions, which might lead to a final positive carbon-emission balance. Besides, the frequent use of conventional metallic-based catalysts causes a significant impact in the environment, reducing the viability and sustainability of such approach in the long term. Overall, a transition towards truly green and sustainable $CO_2$-revalorisation technologies can only be driven by reducing the reaction conditions and developing noble-metal-free catalysts.

Within this context, the synthesis of nitrogen-containing compounds is extremely relevant. More specifically, developing a green process for the synthesis of urea ($CO(NH_2)_2$), a crucial fertilizer and a promising hydrogen carrier, has generated lots of interest. Urea is industrially produced through the Bazarov two-step reaction, starting from $CO_2$ and ammonia ($NH_3$) at a temperature between 170-220°C and at a pressure between 125-250 bar, showing efficiencies of $\leq 10$ %. In these reactions, ammonium carbamate ($[NH_2COO]^-[NH_4]^+$) is initially obtained as a reaction intermediate product and subsequently dehydrated to urea. On the other hand, ammonia ($NH_3$) itself is obtained by means of the Haber-Bosch process (where reaction conditions are 300-500°C and 150-300 bar), resulting in a significantly high carbon footprint. Therefore, the use of photo- and electro-catalysts has been explored to promote the C-N coupling at milder conditions. In this context, most of the studies reported in the literature focus on providing a single-step reaction using $N_2$ or $NO_x$ as nitrogenous reactants to avoid the energetic costs associated with the Haber-Bosch process (i.e. $NH_3$). Such approaches present two main challenges:

1) They need to ensure the activation of the nitrogen source (in addition to the activation of $CO_2$); and
2) They need to provide carefully designed, multifunctional catalysts with several binding acidic/ basic sites for the co-reduction of different species and the promotion of C-N bonds, while avoiding other reaction pathways (such as hydrogen evolution reactions). Indeed, most of the systems explored suffer from low yields and selectivity.

Single-step proposed approaches disclose the development of catalysts with frustrated Lewis pairs or the use of vacancy-rich TiOz composites, effectively reducing the reaction conditions. In addition, apart from the use of complex and expensive catalysts, the proposed single-step catalysed reactions fundamentally differ from the Bazarov reaction, establishing new limiting steps that hinder their industrial viability. It should be noted that, in the Bazarov reaction, the proposed metal complex catalysts were intended to increase the yields rather than moving towards more sustainable reaction conditions. Nevertheless, fewer studies have been reported focusing on the Bazarov reaction.

In view of the above, there is a need to achieve sustainable $CO_2$ revalorisation perspectives that allow efficient $CO_2$ fixation. Focusing specifically on reducing the environmental impact of the Barzarov reaction, it is necessary to promote urea production through strictly mild conditions.

**BRIEF DESCRIPTION OF THE INVENTION**

In response to the above need in the art, the inventors have found that urea may be obtained from carbon dioxide ($CO_2$) and ammonia ($NH_3$) with a selectivity of 97% under strictly mild conditions (e.g., 95-120°C and 1 bar pressure of $CO_2$), in the presence of a permanently polarised hydroxyapatite catalyst, without the need to apply any electrical currents or UV irradiation, which represents an efficiency of approximately 30% with respect to $CO_2$ and approximately 2% with respect to the $NH_3$. In these regards, the addition of $NH_3(I)$ in the aqueous solution of the catalysed reaction facilitates the fixation of $CO_2$, "fixation" being understood as the adsorption and, subsequent, transformation of $CO_2$ into value-added chemical products.

Thus, in a first aspect the invention refers to a method for the synthesis of urea from carbon dioxide ($CO_2$) and ammonia ($NH_3$), wherein the method comprises contacting a gaseous stream of $CO_2$ with an aqueous solution of $NH_3$ in

the presence of a permanently polarised catalyst at a temperature of 70-220°C and at a total gas pressure of 0.01-250 bar.

In a further aspect, the invention refers to the use of a permanently polarised catalyst for:

- reducing $CO_2$ concentration present in a gaseous stream;
- reducing $NH_3$ concentration present in an aqueous solution; and/or
- producing urea,

by implementing a method according to the invention.

## DESCRIPTION OF THE DRAWINGS

The foregoing and other features and advantages will be more fully understood from the detailed description of the invention, as well as from examples referring to the attached figures, which are described in the following paragraphs.

- Figure 1. Structural and morphological characterisation of upp-HAp. (a) WAXD spectrum (the HAp and Bru characteristic reflection are highlighted with squares and circles, respectively), (b) HRTEM pictographs, (c) in-Depth studies by means of Raman microscopy technique (the characteristic Raman modes are highlighted for HAp and Bru, respectively), (d) Bode plot, (e) Representative optical image of the shape and size of the catalysts used; and (f) High magnification SEM image.
- Figure 2. Scheme of the prepared sample pellets for the EIS measurements with respective insets of the EEC for (a) HAp and (b) HAp+TSP.
- Figure 3. Scheme of the reactor used in this study.
- Figure 4. (a) pH dependence on the $NH_3$ content introduced in the reactor (left graph) or on the $NH_3$ molarity (right graph), (b) Zeta ($\zeta$)-potential of HAp powder after the TSP treatment measured at different pH; and (c) stability tests at different temperatures and $NH_3$ content. To do so, the full width at half maximum of the main v1 vibration has been as a structural parameter to detect structural HAp lattice distortion and/or imperfections.
- Figure 5. $NH_3$ absorption studies through XPS analysis. (a) High resolution spectra of N 1s, (b) High resolution spectra of Ca 2p; and (c) High resolution spectra of P 2p. The reference consists of a upp-HAp sample that has not been immersed in liquid $NH_3$.
- Figure 6. $^1$H NMR spectra acquired from the supernatant of the reaction. The presence of EtOH (asterisk) can be only observed at higher magnifications.
- Figure 7. Comparison among the products found adsorbed in the catalyst and in the supernatant.
- Figure 8. Urea efficiency depending on the $NH_3$ initial content with respect (a) $NH_3$ fixation; and (b) $CO_2$ fixation.
- Figure 9. Comparison of $CO_2$ fixation efficiencies obtained for different atmospheres: pure $CO_2$ (left), $CO_2+N_2+NO$ (314 ppm) (centre) and $CO_2+NH_3$ (liquid) (right).
- Figure 10. (a) Bode plot; and (b) $^1$H NMR of the supernatant collected after the reaction with pp-$SiO_2$.

## DETAILED DESCRIPTION OF THE INVENTION

As explained above, the inventors have found that urea may be efficiently and selectively obtained from carbon dioxide ($CO_2$) and ammonia ($NH_3$) under strictly mild conditions (e.g., 95-120°C and 1 bar pressure of $CO_2$) in the presence of a permanently polarised hydroxyapatite catalyst, without the need to apply any electrical currents or UV irradiation.

Thus, in a first aspect the invention refers to a method for the synthesis of urea from carbon dioxide ($CO_2$) and ammonia ($NH_3$), wherein the method comprises contacting a gaseous stream of $CO_2$ with an aqueous solution of $NH_3$ in the presence of a permanently polarised catalyst at a temperature of 70-220°C and at a total gas pressure of 0.01-250 bar.

In embodiments of the invention, the permanently polarised catalyst is a permanently polarised polymeric catalyst or a permanently polarised ceramic catalyst. In particular embodiments, the permanently polarised polymeric catalyst is selected from the group consisting of polyphosphines, metal-functionalised conjugated microporous polymers, metalloporphyrin-based porous ionic polymers, polylactic acid (PLA), polyethylene terephthalate (PET), polyethylene terephthalate glycol (PETG), thermoplastic polyurethane (TPU), poly(3,4-ethylenedioxythiophene) (PEDOT), polyaniline (PANI), polypyrrole (PPy), polystyrene (PS), and combinations thereof. In particular embodiments of the invention, the ceramic material at the basis of the permanently polarised ceramic catalyst is selected from the group consisting of hydroxyapatite ($Ca_5(PO_4)_3(OH)$), alumina ($Al_2O_3$), zirconia ($ZrO_2$), Titania ($TiO_2$), Vanadium(II) oxide (vanadium monoxide, VO), Vanadium(III) oxide (vanadium sesquioxide or trioxide, $V_2O_3$), Vanadium(IV) oxide (vanadium dioxide, $VO_2$), Vanadium(V) oxide (vanadium pentoxide, $V_2O_5$), ferrous (Fe(II)) oxide, ferric (Fe(III)) oxide, silica ($SiO_2$), zeolite (with general formula $M^{n+}_{1/n}(AlO_2)^-(SiO_2)_x \cdot yH_2O$ where $M^{n+}_{1/n}$ is either a metal ion or hydrogen), montmorillonite (($Na,Ca)_{0.3}(Al,Mg)_2Si_4O_{10}(OH)_2 \cdot nH_2O$), $PbTiO_3$, $Pb/Mg_{0.3}Nb_{0.6})O_3$), $BaTiO_3$, Perovskite and combinations thereof. In a particular embodiment, the permanently polarised ceramic catalyst is permanently polarised hydroxyapatite or permanently polarised silicon dioxide (silica).

In certain embodiments, the catalytic materials of the invention may also be decorated with metallic catalytic particles such as Au, Fe, Cu, Mo, Pt, Pd, etc.

In the context of the instant invention, the permanently polarised catalyst can be obtained through a process of thermal stimulated polarisation (TSP), wherein the TSP process comprises the steps of:

a) Applying a constant or variable DC voltage of between 250 V and 2500 V for a minimum of 1 minute at a temperature between 70 °C and 1200 °C, and/or applying an equivalent electric field of between 1.49 k/Vcm and 15 kV/cm for a minimum of 1 minute at a temperature between 35 °C and 1600 °C.

b) Cooling the samples obtained in step (b) while maintaining the electric field or cool the samples obtained in step (b) while maintaining or not maintaining the electrostatic discharge or equivalent electric field.

In the case of permanently polarised ceramic catalysts, the permanently polarised ceramic catalyst can be obtained through a process of thermal stimulated polarisation (TSP), wherein the TSP process comprises the steps of:

a) Sintering the ceramic material at a temperature between 35°C and 1600°C,

b) Applying a constant or variable DC voltage of between 250 V and 2500 V for a minimum of 1 minute at a temperature between 70 °C and 1200 °C, and/or applying an equivalent electric field of between 1.49 k/Vcm and 15 kV/cm for a minimum of 1 minute at a temperature between 35 °C and 1600 °C.

c) Cooling the samples obtained in step (b) while maintaining the electric field or cool the samples obtained in step (b) while maintaining or not maintaining the electrostatic discharge or equivalent electric field.

In a particular embodiment, the thermal stimulated polarisation (TSP) treatment comprises the steps of applying a constant 500 V at 1000°C for 1 hour, wherein the ceramic catalyst samples are placed between two stainless steel plates, acting as electrodes, the samples are left in contact with the negative electrode, and the positive electrode is separated 4 cm. The samples thus treated are allowed to cool down while maintaining the applied voltage for 30 minutes. The TSP process, capable of inducing a permanently polarised state, is carried out at a temperature that avoids any crystallographic phase transitions. This temperature will be different for different materials. For instance, this temperature may be of 1000°C for hydroxyapatite or of 500°C for silicon dioxide. Methods to carry out thermal stimulated polarisation are known to the skilled person (please see WO2018024727 A1, WO2023139032 A1, and Jordi Sans et al., "Synthesis of urea from CO2 and N2 fixation under mild conditions using polarised hydroxyapatite as a catalyst", Sustainable Energy Fuels, 2024, 8, 1473). Any suitable method for the production of a permanently polarised ceramic catalyst may be used in the context of the present invention.

The catalytic material transformed by means of the TSP treatment (such that it becomes permanently polarised) acquires improved structural and electrical properties that are maintained over time. Amongst these properties, the materials show:

- the presence of different <u>acidic and/or basic binding sites</u> that favour the simultaneous adsorption of $CO_2$ and ammonia ($NH_3$).
- an improvement in the <u>electrical conductivity</u> of the bulk material by 2 to 1000 times, preferably by 5 to 500 times, more preferably by 10 to 100 times.
- an improvement in <u>surface charge accumulation</u> from 1 to 100 times, preferably from 2 to 50 times, more preferably from 2 to 10 times. In particular embodiments of the invention, the improvement in surface charge accumulation is increased and/or stabilized in a pH range of 6 to 14, preferably from 7 to 14, more preferably from 8 to 12.

In embodiments of the invention, the catalyst can be prepared as a compact solid or it can be porous. Thus, in a particular embodiment, the permanently polarised catalyst is a non-porous material or a porous material. As used herein, a material is considered to be non-porous when it has a porosity of $\leq$ 1000 g/L when using, as a determination method, the BJH method in accordance with DIN 66134 (Mesopore analysis by nitrogen sorption using the method of Barrett, Joyner and Halenda (BJH)). In particular embodiments, the material is a porous material and has a mean pore diameter in between 1 nm to 500.000 nm, when determined by means of Scanning Electron Microscopy. In the context of the invention, the pores may have any shape or form. In a particular embodiment, the pores are preferably in the form of spherical pores. In a particular embodiment, the pores are interconnected in at least 70, at least 80, or at least 90% of the total volume of the pores.

In the catalysed chemical reaction of the invention, urea is obtained from carbon dioxide and ammonia. Carbon dioxide ($CO_2$) is provided as a gaseous stream. In particular embodiments of the invention, carbon dioxide ($CO_2$) makes up at least 20%, at least 30%, at least 40%, at least 50%, at least 60%, at least 70%, at least 80%, at least 85%, at least 90%, at least 95%, at least 98%, or at least 99% of the gaseous stream. In a particular embodiment, carbon dioxide ($CO_2$) makes up

100% of the gaseous stream (i.e., a pure, undiluted $CO_2$ stream). In embodiments of the invention, the $CO_2$ pressure is between 0.01-250 bar, between 1-6 bar, or between 1-3 bar. In particular embodiments, the $CO_2$ pressure is 0.2, 0.4, 0.6, 0.8, 1.0, 1.5, 2.0, 2.5, 3.0, 4.0, 5.0, or 6.0 bar. Ammonia ($NH_3$) is provided as an aqueous solution. In embodiments of the invention, the $NH_3$ concentration in the aqueous solution is between $0.5 \times 10^{-3}$ - 100.0 M, or between $1.0 \times 10^{-3}$-20.0 M. In particular embodiments, the $NH_3$ concentration in the aqueous solution is $0.5 \times 10^{-3}$M, $1.0 \times 10^{-3}$M, $1.0 \times 10^{-2}$M, $1.0 \times 10^{-1}$M, 1.0 M, 2.0 M, 3.0 M, 4.0 M, 5.0 M, 10 M, 50 M, or 100 M. In particular embodiments of the invention, the volumetric ratio of aqueous solution to catalyst is between 10000:1 and 0.1:1, preferably between 1000:1 and 1:1, more preferably between 1000:1 to 100:1. In particular embodiments of the invention, the volumetric ratio of gaseous $CO_2$ to aqueous solution is between 100:1 and 1:100, preferably between 10:1 and 1:100, more preferably between 5:1 to 1:10.

As indicated above, the catalysed chemical reaction of the invention is carried out at a temperature of 70-220°C and at a total gas pressure of 0.01-250 bar. In embodiments of the invention, the temperature is between 70 and 220°C, preferably between 80 and 150°C, more preferably between 90 and 140°C. In particular embodiments of the invention, the catalysed chemical reaction of the invention is carried out at a temperature of 70, 80, 90, 100, 110, 120, 130, 140, 150, 160, 170, 180, 190, 200, or 220 °C. In a particular embodiment, the catalysed chemical reaction of the invention is carried out at a temperature of 120°C. Also, in embodiments of the invention, the total gas pressure is between 0.01 and 250 bar, preferably between 0.1 and 20 bar, or 0.8 and 10 bar, more preferably between 1 and 6 bar. In particular embodiments of the invention, the catalysed chemical reaction of the invention is carried out at a total gas pressure of 0.01, 0.05, 0.1, 0.2, 0.4, 0.6, 0.8, 1.0, 1.5, 2.0, 2.5, 3.0, 4.0, 5.0, 6.0, 7.0, 8.0, 9.0, 10.0, 12.0, 15.0, 20.0, 40.0, 80.0, 120.0, 160.0, 200.0, or 250.0 bar. In the context of the invention, the term "total gas pressure" refers to the gas pressure of $CO_2$ and any other accompanying gas that may be present in the gas stream. In streams of pure $CO_2$, wherein the gas composition is 100% $CO_2$, the term "total gas pressure" is equivalent to "$CO_2$ pressure". In particular embodiments of the invention, the reaction time is between 10 minutes to 48 hours, preferably between 6 hours to 42 hours, more preferably between 18 hours to 36 hours in the case of batch reactions. In alternative embodiments, gas flow rates are from 0.01 to 1000 mL/s in the case of continuous reactions.

AQUI

An advantage of the method of the present invention for the synthesis of urea from carbon dioxide ($CO_2$) and ammonia ($NH_3$) in the presence of the permanently polarised ceramic catalyst is that the efficiency of $CO_2$ fixation is increased. $CO_2$ fixation is the yield measured from the moles of $CO_2$ that have been converted into a product: urea, ethanol, methanol, etc. Thus, the term "$CO_2$ fixation" refers to the percentages of fixation on the active sites of the catalyst (i.e. adsorption), such that, the more $CO_2$ gets adsorbed onto the active sites of the catalyst, the higher yield of conversion of $CO_2$ into a product. In the case of the instant invention, the addition of $NH_3$(l) to the aqueous medium, results in more efficient $CO_2$ adsorption and, consequently, higher $CO_2$ fixation and higher yields of the final product. In the context of the instant invention, introducing ammonia ($NH_3$) into the $CO_2$ fixation reaction increases their respective efficiencies (i.e., $CO_2$ fixation efficiency and $NH_3$ fixation efficiency), since it promotes:

- Reaction conditions at pH values between 7-14. In particular embodiments, the pH values are between, 7-14, or between 8-12. In embodiments of the invention, the pH values are 7, 8, 9, 10, 11, 12, 13, or 14. These basic pH values result in increased catalytic activity of the permanently polarized catalyst. In fact, the introduction of $NH_3$ prevents the acidification of the water due to the presence of $CO_2$, so that pH values in the range of 5 to 7, in particular 6 to 7 (a range where the permanently polarized catalyst is reduced in its catalytic activity) are avoided.
- Kinetic constants for the generation of C-N bonds that are more favourable than the kinetic constants for the generation of C-C bonds.
- The generation of methyl carbamate ($H_2NCOOCH_3$) as an intermediate of the reaction, which presents a kinetic constant that is more favourable than other nitrogenous and/or carbonated intermediates such as ammonium carbamate ($[H_2NCOO]^-[NH_4]^+$).
- The generation of nitrogenous end products (i.e., urea) that present a more favourable desorption kinetic constant than non-nitrogenous end products.

In regards the $CO_2$ fixation efficiency, said $CO_2$ fixation efficiency to produce urea is, in embodiments of the invention, > 3%, preferably > 10%, more preferably > 30%, when urea is produced from $CO_2$ and ammonia ($NH_3$) in the presence of a polarised ceramic catalyst. In contrast, the urea production efficiency is < 10%, preferably < 5%, more preferably < 1%, in the absence of ammonia ($NH_3$). In regards the $NH_3$ fixation efficiency, said $NH_3$ fixation efficiency with respect to urea generation is, in embodiments of the invention, > 1%, preferably > 1.5%.

The catalysed chemical reaction of the present invention results in the production of urea ($CO(NH_2)_2$) with a high degree of selectivity. In embodiments of the invention, the selectivity of the reaction towards urea is in excess of 75%, in excess of 80%, in excess of 85%, in excess of 90%, in excess of 95%, in excess of 97%, or in excess of 99%. In the context of the invention, urea is readily desorbed from the catalyst as the catalysed chemical reaction progresses. In embodiments of the invention, the total percentage of urea that is desorbed from the catalyst at the end of the process is at least 70%, at least 80%, at least 90%, at least 95%, at least 98% or at least 99%. In an embodiment, the total percentage of urea that is desorbed from the catalyst at the end of the process is 100%. In embodiments of the invention, the percentage of urea with respect to other carbon by-products that are desorbed from the catalyst at the end of the process is at least 70%, at least 80%, at least 90%, at least 95%, at least 98% or at least 99%.

In particular embodiments, the catalysed reaction for the synthesis of urea from carbon dioxide ($CO_2$) and ammonia ($NH_3$) according to the invention is carried out under UV irradiation and/or assisted by applying external electrical currents.

In another aspect, the invention refers to the use of a permanently polarised catalyst for:

- reducing $CO_2$ concentration present in a gaseous stream;
- reducing $NH_3$ concentration present in an aqueous solution; and/or
- producing urea,

by implementing a method according to the instant invention.

In particular uses of the invention, the catalysed chemical reaction may be used with the primary aim to reduce the $CO_2$ content which may be present in the atmosphere or in polluted air. In other uses, the main intention may be to reduce the ammonia ($NH_3$) content in water, particularly from contaminated water. Finally, in particular uses of the invention, the main intention may be to simply produce urea as a value-added product by itself.

All the terms and embodiments described anywhere in this document are equally applicable to all aspects of the invention. It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice. Furthermore, any of the parametric ranges and/or single values of temperature (T), pressure (P), etc. described in the examples can be used, in different embodiments of the invention, in isolation or in combination.

**EXAMPLES**

The following invention is hereby described by way of the following examples, which are to be construed as merely illustrative and not limitative of the scope of the invention.

**Example 1 - Design and characterisation of the catalyst**

*1.1 Rationale*

HAp has been reported as a catalyst for a variety of chemical reactions. In particular, HAp has shown outstanding catalytic activity towards highly stable molecules (e.g. $N_2$ or $CO_2$) when it becomes permanently polarised (pp-HAp) by a thermally stimulated polarisation (TSP) treatment. Such behaviour has been directly attributed to both: (a) generation of $OH^-$ vacancies and permanent re-orientation of hydroxyl groups contained within the lattice, which are aligned in columns, globally pointing to a specific direction; and (b) intensive lattice refinement showing superior crystallinity and large crystallite size domains. Thus, pp-HAp presents much higher electrical charge transport properties (up to two orders of magnitude). In addition, pp-HAp has been successfully shaped into ultraporous cubes (upp-HAp) to increase the exposed surface area without compromising the aforementioned lattice structure and its resulting enhanced electrical properties. Although TSP treatment has also been proposed for other materials, exhaustive studies have been published focusing specifically on upp-HAp due to its catalytic relevance. Such works settle down the fundaments of the TSP treatment by establishing appropriate characterisation protocols to determine the optimal experimental conditions and mechanisms to enhance the catalytic activity of upp-HAp.

*1.2 Synthesis of upp-HAp*

Hydroxyapatite (HAp) powder was synthesized following a hydrothermal route described elsewhere (please see WO2023139032 A1). Ultraporous HAp scaffolds were obtained by mixing 40% wt. of grinded HAp fine powder with 60% wt. of Pluronic® F-127 hydrogel in a cold room (4 °C) which was added dropwise. Accordingly, Pluronic® F-127 hydrogel was prepared by mixing 25 g of distilled water with 25g of Plutonic® F-127 polymer using a FlackTek Speed Mixer at 3500 rpm for 5 minutes. Then, 50 g of Pluronic® F-127 polymer were additionally added repeating the process. To achieve a homogeneous HAp/ hydrogel mixture, such blend was periodically stirred at 2500 rpm for 2 minutes using a Fisherbrand™ Digital Vortex Mixer. The resulting inks were viscous enough to allow 3D modelling using previously cooled spatulas (< 4°C). The HAp scaffolds obtained were calcined at 1000 °C for 1 h using a Carbolite ELF11/6B/301 muffle. After the calcination treatment, the Pluronic® F-127 hydrogel was completely removed leading to a highly porous HAp structure. Finally, the calcined HAp scaffolds were submitted to a thermal stimulated polarisation (TSP) treatment consisting of applying a constant 500 V (GAMMA power supply) at 1000 °C (ELF11/6B/301 adapted muffle) for 1 h. To do so, samples were placed between two stainless steel plates (AISI 304), acting as electrodes. The sample was left in contact with the negative electrode. Conversely, the positive electrode was separated 4 cm. The resulting upp-HAp samples were allowed to cool down maintaining the applied voltage for 30 minutes.

### 1.3 Characterisation of the catalyst

The successful polarisation of upp-HAp (responsible for the high catalytic activity) was confirmed through previously established protocols extensively reported in the literature.

Structural analyses were performed by means of a inVia Qontor confocal Raman microscope (Renishaw) equipped with a Renishaw Centrus 2957T2 detector and a 532 nm laser. In order to obtain representative data, 32 single point spectra were averaged. In addition, Wide angle X-ray diffraction (WAXD) studies were conducted using a Brucker D8 Advance model with Bragg-Brentano $2\theta$ configuration and Cu $K_\alpha$ radiation ($\lambda$ = 0.1542 nm). Measurements were performed in a $2\theta$ range of 20°-60° in steps of 0.02° and scan speed of 2 s, using a one-dimensional Lynx Eye detector. The crystallinity ($\chi_c$) was obtained using the following expression:

$$\chi_c = 1 - \frac{V_{112/300}}{I_{300}} \qquad \text{(Eq. 1)}$$

where $I_{300}$ is the intensity of the (300) reflection and $V_{112/300}$ is the intensity of the hollow between the (112) and (300) reflections, which disappears in non-crystalline samples. Further structural insights were acquired through high-resolution transmission electron microscopy (HRTEM) performed in a JEOL 2010F microscope operating with a field emission electron source at 200 kV.

The porosity of the upp-HAp catalysts and morphological characterisation was obtained averaging five different scanning electron microscopy (SEM) images collected using a Focused Ion Beam Zeiss Neon40 microscope equipped with a SEM GEMINI column with a Shottky field emission. Samples were sputter-coated with a thin layer of carbon to prevent sample charging problems. Moreover, upp-HAp was analysed using X-ray photoelectron spectroscopy (XPS) on a SPECS system. The spectrometer was equipped with a high-intensity twin-anode X-ray source XR50 of Mg/Al (1253 eV/ 1487 eV) operating with the Al anode at 150 W, placed perpendicular to the analyser axis, and using a Phoibos 150 MCD-9 XP detector. The position of the stage was digitally controlled to ensure that the spot was in the same exact place during the whole treatment. The pass energy of the hemispherical analyser was set at 25 eV and the energy step of high-resolution spectra was set at 0.1 eV. The pressure in the analysis chamber was always below $10^{-7}$ Pa, and binding energy (BE) values were referred to the C 1s peak at 284.8 eV.

HAp and HAp+TSP pellet samples were analysed by means of electrochemical impedance spectroscopy (EIS). EIS studies performed using a VIONIC potentiostat from Metrohm connected to a conductivity meter cell through two stainless steel electrodes AISI 304 supported by a teflon holder. Due to the high sensitivity of the equipment, measurements were performed without electrolyte solution, in the 1 MHz - 10 mHz frequency range and applying a 100 mV sinusoidal voltage. Electrical characterisation was performed through electrochemical impedance spectra (EIS) technique (VIONIC autolab from Metrohm) and Zeta ($\zeta$)-potential measurements conducted in a NanoBrook 90Plus Zeta equipped with an AQ.1321 cell. The measurements were acquired suspending 1.5 mg of HAp in 5 mL of a 1 mM KCl water solution, previously sonicated for 3 minutes. The pH of the solution was controlled with $H_2SO_4$ and NaOH using a Crison pH meter GLP-21. The EECs parameters were obtained from fitting the EIS data to EECs previously designed according to the bulk layers of the material (reported elsewhere).

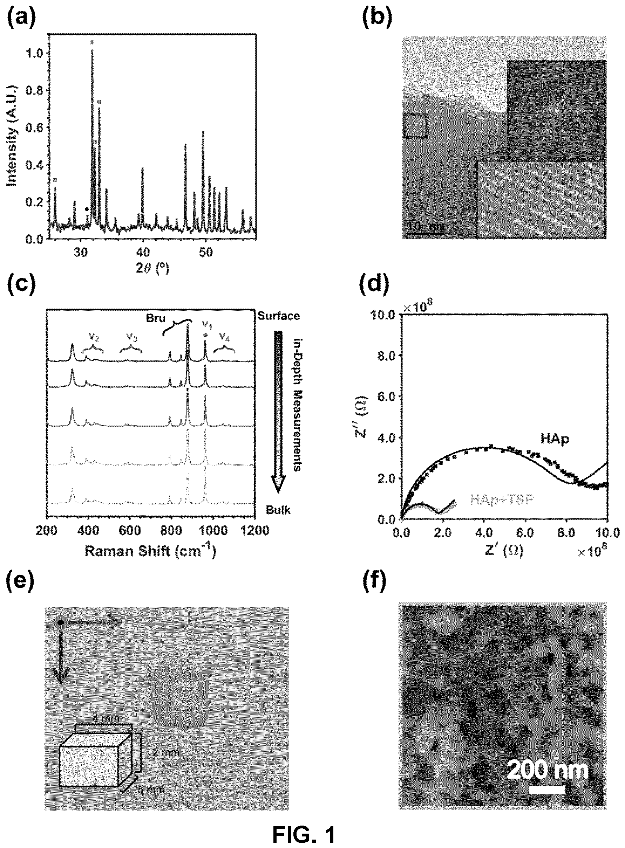

Accordingly, the structural, morphological and electrical response fingerprints of upp-HAp are presented in Figure 1.

As it can be seen in Figure 1a, the WAXD spectrum of upp-HAp presents the characteristic reflections of HAp at $2\theta$ = 25.9°, 31.8°, 32.3°, 33.0° attributed to the (002), (211), (112), (300) crystallographic planes, respectively. Moreover, the crystallinity ($\chi_c$), obtained from the main (211) reflection (see equation Eq. 1, above), is $0.94 \pm 0.02$ being in complete agreement with the $\chi_c$ values reported for other pp-HAp systems (i.e. $\chi_c$ > 0.90) (J. Sans, et al., "Hydroxyapatite-based biphasic catalysts with plasticity properties and its potential in carbon dioxide fixation", Chem. Eng. J. 2022, 433, 133512). The extremely high crystallinity of upp-HAp can be also observed in the acquired HRTEM pictographs (Figure 1b) which reveal the existence of large crystallographic domains as seen by the well-defined lattice fringes at 3.1 Å and 3.4 Å (see inset in Figure 1b). Such spots correspond to the (210) and (002) crystallographic planes, respectively, supporting the previous WAXD results. Besides, the characteristic superstructure attributed to the permanent polarised state at 6.9 Å (001) is also observed. Finally, the defined carved surface discards the presence of amorphous calcium phosphate (ACP) at the interface of the particles, which has been shown to clearly restrict the electrical transport properties of HAp. The in-depth Raman spectra depicted in Figure 1c allows the unambiguous determination of the co-existence of other calcium phosphate salts at both the surface and at the bulk of the sample. Unlike the WAXD reflections which tend to superpose for different calcium phosphate salts, their Raman spectra are mainly governed by the vibrations of the $PO_4^{3-}$ tetrahedra which can split or shift depending on the $PO_4^{3-}$ different crystallographic sites or the presence of distortions. Correspondingly, the characteristic Raman vibration modes of HAp can be found at $v_1$ = 962 cm$^{-1}$ (P-O symmetric stretching), $v_2$= 400-480 cm$^{-1}$ (doubly degenerated O-P-O bending), $v_3$ = 570-625 cm$^{-1}$ (P-O triply degenerated asymmetric stretching) and $v_4$ = 1020-1095 cm$^{-1}$ (triply degenerated O-P-O bending mode). Accordingly, the sharp $v_1$ peak allows discarding the presence of representative amounts of known catalytic interfering phases like ACP or β-tricalcium phosphate (βTCP) throughout the sample. Certainly, the peaks located at 878, 844 and 794 cm$^{-1}$ (black circles) confirm the presence of brushite (Bru, $CaHPO_4$) stabilized at the surface of the upp-HAp sample as a result of the TSP treatment (i.e. note how the intensity of such peaks decreases when increasing the depth of the measurement). Bru can be further confirmed in the Figure 1a for its characteristic reflections at 2θ = 31.1 ° (121) and some other superposed peaks (see black circles). The presence of Bru at the surface is advantageous for both the catalytic synergistic effect (i.e. plasticity behaviour on the selectivity) and because it can be further used as a quality control to ensure that the TSP treatment has been applied successfully.

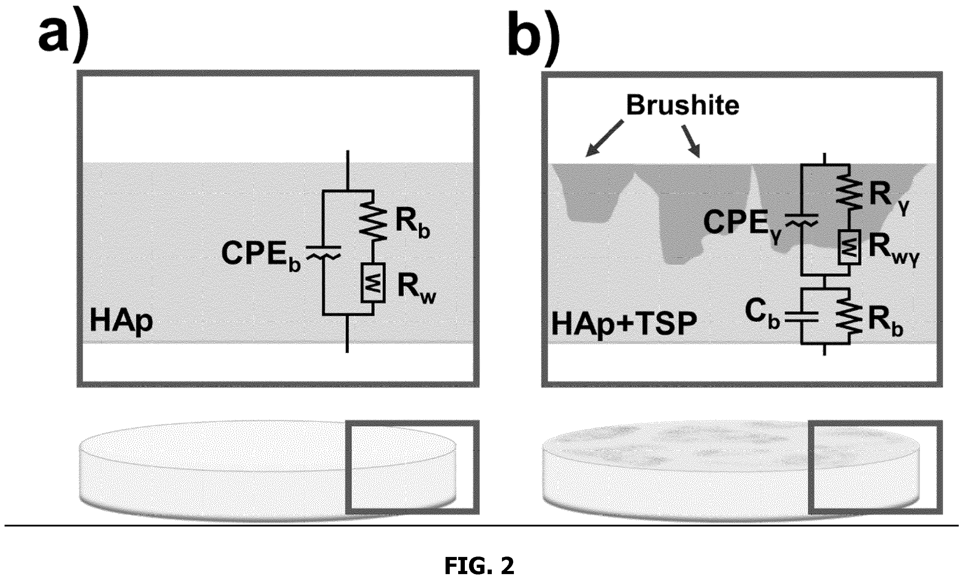

The enhanced electrical response of upp-HAp compared to sintered HAp was assessed by means of the EIS technique. As it can be observed in Figure 2a, HAp presented an electrical response which was modelled using a Randles circuit. However, upon polarising the sample (HAp+TSP), formation of the Bru surficial layer and the HAp hydroxyl groups re-orientation re-shaped the initial EEC into a more complex model, Figure 2b. The Bru layer was modelled as a Randles circuit connected in series to the new EEC of the polarised HAp, the latter consisting of a parallel connected resistor and capacitor resembling a conductor material EEC. As shown in the Bode plot of Figure 1d, as soon as the TSP treatment is applied the semi-circle response, attributed to a semi-conductor behaviour, decreases drastically. Correspondingly, the electrical resistance of the bulk ($R_b$) derived from fitting the experimental data (see the modelling details of the equivalent electrical circuit at the ESI) changes from 569 ± 11 Ω·cm$^{-2}$ to 10 ± 0.5 Ω·cm$^{-2}$ for HAp and pp-HAp, respectively. The complete electrical values obtained with their associated fitting errors are presented in Table 1.

Table 1. Data derived from the fitting of the electrochemical impedance spectra recorded for dry HAp before and after the TSP treatment. The modelling of the electrical equivalent circuit (EEC) is explained above. The statistical error associated to each circuit element is displayed in parenthesis (in %). CPE accounts for a constant phase element.

| EEC Model | HAp before the TSP treatment | HAp after the TSP treatment |
|---|---|---|
| $R_b$ (MΩ cm$^{-2}$) | 568.66 (2.60%) | 9.65 (5.88%) |
| $CPE_b$ (pF·cm$^{-2}$·s$^{n-1}$) | 20.61 (2.93%) | - |
| $n_b$ | 0.93 (0.29%) | - |
| $R_w$ (nF·cm$^{-2}$·s$^{-1/2}$) | 2.44 (10.26%) | - |
| $R_\gamma$ (MΩ cm$^{-2}$) | - | 114.48 (0.88%) |
| $CPE_\gamma$ (pF·cm$^{-2}$·s$^{n-1}$) | - | 23.89 (2.65%) |
| $n_\gamma$ | - | 0.96 (0.26%) |
| $R_{w\gamma}$ (nF·cm$^{-2}$·s$^{-1/2}$) | - | 7.11 (2.54%) |
| $C_b$ (pF·cm$^{-2}$) | - | 20.7 (3.14%) |

Finally, the catalysts were shaped into porous cubes of $4 \times 5 \times 2$ mm$^3$ (Figure 1e) showing a porosity of 16 % with a pore size of 142 ± 37 nm as determined by SEM inspection in Figure 1f. Overall, the exhaustive characterisation presented ensures the proper preparation of upp-HAp as a catalyst.

## Example 2 - Synthesis of urea from $CO_2$ and $NH_3$

### 2.1 Experimental set-up

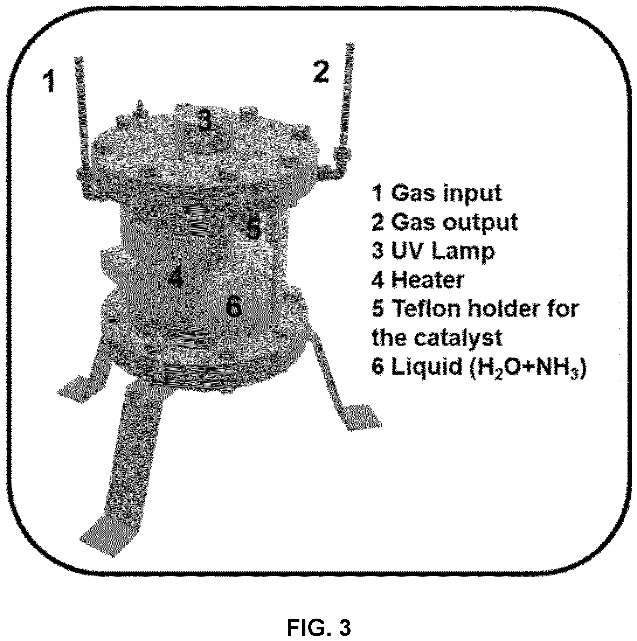

As depicted in Figure 3, a customised batch reactor coated with a perfluorinated polymer (120 mL) has been considered in this study. $CO_2$ (4X, purity of 99.998 %) was purchased from Nippon Gases. $NH_3$ (purity of 20 %) was purchased from PanReac AppliChem. The reactor was equipped with a UV lamp (GPH265T5U4, λ = 253.7 nm) placed in the middle of the reactor (irradiating directly the catalyst) and protected with a UV transparent quartz tube. For the reaction a vacuum pump was used to eliminate the initial air content of the reaction chamber. The synthesis of urea from $CO_2$ and $NH_3$ (the present invention) was carried out using the same reactor. Reactions were conducted at 1 bar of $CO_2$ and in the 70-120 °C range for 24 h and without applying UV irradiation. 0.2-2 mL of $NH_3$ were mixed in water to obtain a final constant liquid volume of 44.8 mL.

The reaction products found absorbed in the upp-HAp catalyst and dissolved in water after the reactions (hereafter referred as "supernatant") were analysed by $^1$H NMR spectroscopy (Brucker Ascend 400 operating at 400 MHz) recording 256 scans and using tetramethylsilane as an internal standard for the chemical shift ($\delta$) calibration. In order to favour the desorption of the products, all $^1$H NMR spectra were acquired under acidic conditions (pH = 2.1 $\pm$ 0.2) using 7.6 mM of $H_2SO_4$ and following optimised acquisition protocols (i.e. DMSO-$d_6$ as the deuterated solvent, calibration of the $\delta$ shift of urea depending on the pH and water suppression methods) described in the ESI and in the literature. Finally, the yield of the products was calculated by integrating the areas of the peaks normalized by the contribution of protons and calibrated through external references. The final yield reported was normalized per gram of catalyst ($g_{cat}$). The selectivity was calculated as the ratio between the urea and the sum of the yields of all the products detected. The efficiencies were calculated from the initial moles of $CO_2$ and $NH_3$ introduced in the reactor, respectively.

*2.2 Suitability of upp-HAp for Bazarov reactions*

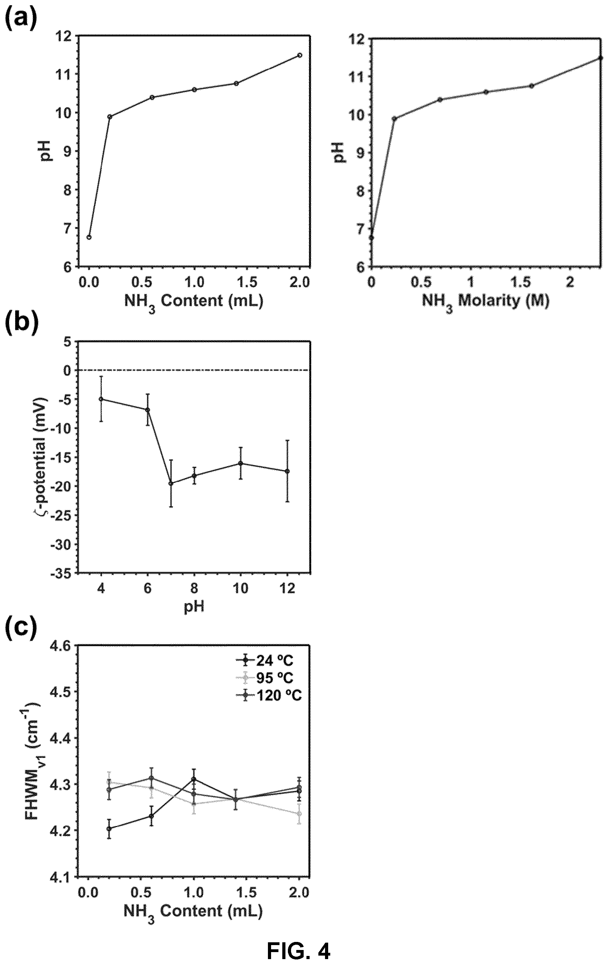

While for most of the reported studies the water content introduced in the reactor has a pH close to 7, the Bazarov reaction intrinsically occurs at pH > 7 due to the presence of liquid $NH_3$. Since the catalytic activity of upp-HAp strongly relies on its electrical properties, studying how the surface charge accumulation varies depending on the pH becomes essential. To do so, the change of pH depending on the $NH_3$ content presented in Figure 4a (left graph) has been considered as a reference for measuring the Zeta ($\zeta$)-potential of upp-HAp. The equivalent pH dependence on the molarity is presented in Figure 4a (right graph). As it can be seen in Figure 4b, while the point of zero charge appears to fall into strong acid media (not seen), for pH $\geq$ 7 the ($\zeta$)-potential of upp-HAp stabilises within the [-14, -20] mV range. Interestingly, a clear decrease in the ($\zeta$)-potential is observed for pH < 7. As compared to other heterogeneous reactions reported, the presence of $NH_3$ could be advantageous for the catalytic activity of upp-HAp. That is, while the presence of wet $CO_2$ tends to acidify the water, potentially reaching a ($\zeta$)-potential region with far less charge accumulation, the liquid $NH_3$ ensures that the reaction is strictly performed at a pH > 7 were the absolute value of ($\zeta$)-potential is maximum.

Even though HAp does not dissolve under moderate basic media, stability tests of the catalyst were carried out to determine whether the crystalline refinement is maintained under reaction conditions. For this reason, upp-HAp was immersed in different $NH_3$ solutions and exposed to different temperature cycles. According to the characterisation discussion presented above, the HAp Raman vibration mode $v_1$ was used to discard the presence of other calcium phosphate salts as degradation products (not shown). Moreover, the presence of distortion was studied from the full width half maximum of $v_1$ ($FWHM_{v1}$). As it has been depicted in Figure 4c, the $FWHM_{v1}$ does not change significantly in any of the reaction conditions showing an excellent stability.

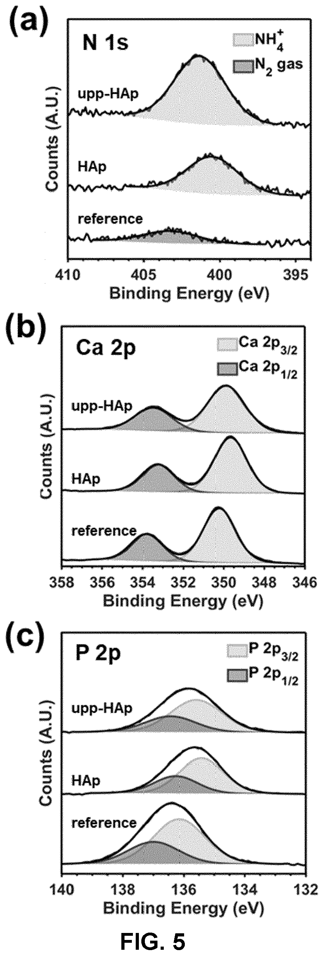

On the other hand, the multifunctional nature of upp-HAp catalysts capable of performing both $N_2$ and $CO_2$ fixation reactions has also been tested considering $NH_3$. Certainly, upp-HAp presents several acid sites located at the $Ca^{2+}$ (Lewis acid site, most active), $OH^-$ vacancy and P-OH (Brønsted irreversible site; attributed to the brushite (Bru) at the interface). Contrarily, upp-HAp also has two main basic sites located at the $PO_4^{3-}$ tetrahedra and the OH- groups. Thus, the absorption of $NH_3$ on the acid binding sites of upp-HAp has been studied through XPS analyses (Figure 5). More specifically, HAp (i.e. without applying the TSP treatment) and upp-HAp samples were immersed into $NH_3$ for 24 h and subsequently dried at 30 °C for 24 h. Therefore, only the chemisorbed $NH_3$ remained attached to the acid binding sites. Note also that the XPS measurements were performed under ultra-high vacuum conditions. Besides, upp-HAp (as prepared) has been used as a reference.

Figure 5a depicts the high resolution XPS spectra of the N 1s the region. As it can be observed, while the reference shows a small peak at 403.3 eV corresponding to absorbed $N_2$ gas, both HAp and upp-HAp samples immersed to $NH_3$ present a clear peak at 400.5 eV and 401.3 eV, respectively; attributed to absorbed ammonium ($NH_4^+$). Interestingly, HAp shows a lower binding energy which is closer to the $NH_3$ (399-400 eV) highlighting the superior catalytic activity of the binding sites of upp-HAp. Moreover, the positive effect of the TSP treatment of upp-HAp is also observed in the total amount of $NH_3$ absorbed in the lattice, which has been derived from the N 1s / Ca 2p ratio. Accordingly, the absorption ratio of upp-HAp is of 0.47 and almost doubles the $NH_3$ absorption capacity of HAp (0.26). The ratio obtained for the $N_2$ absorption of the reference is of 0.09. Finally, the high resolution XPS spectra of the Ca 2p and P 2p regions are presented in Figures 5b and 5c. Accordingly, both species present a shift of approximately -0.4 eV and -0.5 eV for Ca 2p (peaks at 349.8 eV Ca $2p_{3/2}$ and 353.4 eV Ca $2p_{1/2}$) and P 2p (peaks at 135.5 eV P $2p_{3/2}$ and 136.5 eV P $2p_{1/2}$) respectively, with respect to the reference (350.2 eV Ca $2p_{3/2}$ and 353.8 eV Ca $2p_{1/2}$; 136.1 eV P $2p_{3/2}$ and 137.0 P $2p_{1/2}$); attributed to the anchoring of $NH_3$ to the acid binding sites.

Summing up, upp-HAp can be considered a suitable catalyst for the Bazarov reaction, showing great potential (considering both the stability and the catalytic activity) for becoming a green and sustainable alternative to the current catalysts with the associated reaction conditions used.

*2.3 Synthesis of urea*

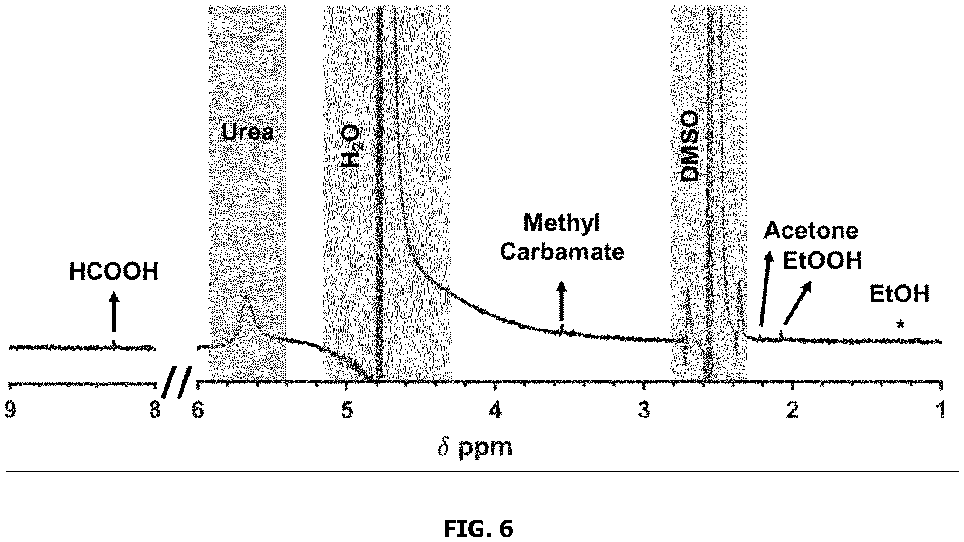

Figure 6 shows $^1$H NMR spectra acquired from the supernatant of the reaction, collected after a reaction was carried out at 120 °C, 1 bar of $CO_2$ and 1 mL of $NH_3$, for 24 h. A blank reaction (i.e. same reaction conditions but without using upp-HAp as catalyst) was also acquired (not sho9wn). As it can be observed, the spectrum reveals a prominent peak located at the characteristic chemical shift ($\delta$) of urea at 5.67 ppm, showing a production yield of $0.32 \pm 0.02$ mmol/$g_{cat}$. As expected, other carbon products associated with the $CO_2$ fixation have been also detected at 1.09 ppm (triplet of ethanol; EtOH), 2.07 ppm (acetic acid; EtOOH), 2.22 ppm (acetone) and 8.29 ppm (formic acid; HCOOH). Nonetheless their yields are extremely low compared with the production of urea ($1.57 \pm 0.11$ $\mu$mol/$g_{cat}$, $3.28 \pm 0.26$ $\mu$mol/$g_{cat}$, $0.51 \pm 0.03$ $\mu$mol/$g_{cat}$ and $8.85 \pm 0.58$ $\mu$mol/$g_{cat}$; respectively), resulting in a urea selectivity higher than 95 %. Accordingly, such results represent a urea production efficiency of 1.19 % and 9.82 % with respect $NH_3$ and $CO_2$, respectively.

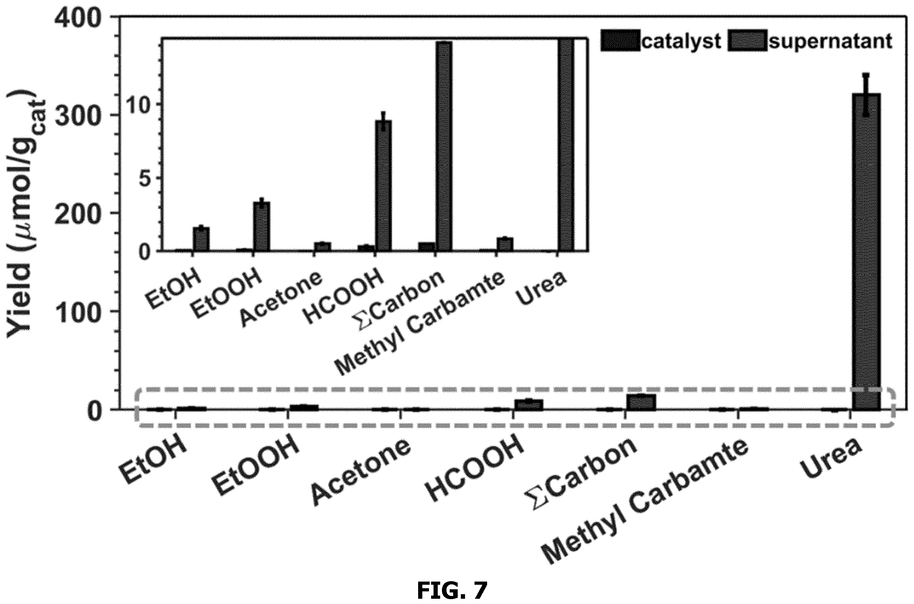

The analysis of the products adsorbed in the catalyst compared with the supernatant depicted in Figure 7 confirms that urea is completely desorbed from the catalyst, representing a great advantage for both the scalability and the industrial feasibility of the process (i.e. avoids catalyst poisoning and favours the recovery of the product). According to previous results (Jordi Sans et al., "Synthesis of urea from CO2 and N2 fixation under mild conditions using polarised hydroxyapatite as a catalyst", Sustainable Energy Fuels, 2024, 8, 1473), methyl carbamate is found as an intermediate ($\delta$ = 3.55 ppm) in both the supernatant and the catalyst ($0.86 \pm 0.04$ $\mu$mol/$g_{cat}$ and $0.06 \pm 0.01$ $\mu$mol/$g_{cat}$; respectively). Interestingly, such intermediate slightly differs from the expected ammonium carbamate intermediate, characteristic of Bazarov industrial reaction. Therefore, while in the industrial Bazarov reaction the rate determining step is governed by the dehydration of carbamate to form urea due to the kinetic barriers associated to the generation of the second C-N bond, the higher reactivity of the methoxy group ($-OCH_3$) on the surface of upp-HAp favours the significant reduction of the energetic conditions of the reaction. Accordingly, the yields of methyl carbamate in Figure 7 are, in general, lower than the rest of the carbon products; suggesting that the limiting step relies on the generation of the first C-N bond after the initial $CO_2$ activation.

*2.4 Effect of $NH_3$ concentration*

In order to obtain further insights on the kinetics of the reaction and potential of the catalyst, several reactions considering the same temperature, time and $CO_2$ pressure conditions but with different $NH_3$ concentrations (0.2 mL, 1 mL and 2 mL) were considered. In Table 2 are presented the yields obtained for each reaction.

**Table 2.** Yields (in $\mu$mol/$g_{cat}$) of the reactions conducted at 120 °C, 1 bar $CO_2$ and 24 h, for different $NH_3$ volumes in the 120 mL reactor.

| | | EtOH | EtOOH | Acetone | HCOOH | ΣCarbon Products | Methyl Carbamate | Urea (mmol/$g_{cat}$) |
|---|---|---|---|---|---|---|---|---|
| **0.2 mL of $NH_3$** | | | | | | | | |
| Catalyst | | 1.88 ±0.26 | 0.83 ±0.19 | 0.10 ±0.03 | 5.40 ±0.91 | 8.22 | 1.64 ±0.34 | 0 |
| Super natant | | 57.76 ±2.71 | 65.60 ±5.25 | 28.73 ±3.11 | 99.59 ±7.81 | 251.68 | 54.86 ±2.74 | 0.12 ±0.02 |
| **1 mL of $NH_3$** | | | | | | | | |
| Catalyst | | 0.06 ±0.01 | 0.10 ±0.02 | 0.01 ±0.005 | 0.32 ±0.07 | 0.51 | 0.06 ±0.01 | 0 |
| Super natant | | 1.57 ±0.11 | 3.28 ±0.26 | 0.51 ±0.03 | 8.85 ±0.58 | 14.21 | 0.86 ±0.04 | 0.32 ±0.02 |
| **2 mL of $NH_3$** | | | | | | | | |
| Catalyst | | 0.05 ±0.01 | 0.05 ±0.01 | 0.09 ±0.02 | 0.42 ±0.07 | 0.61 | 0.39 ±0.03 | 0 |
| Super natant | | 2.77 ±0.28 | 7.92 ±0.95 | 1.28 ±0.26 | 16.62 ±2.16 | 28.59 | 3.46 ±0.31 | 1.01 ±0.13 |

As it can be observed, when the lower quantity of $NH_3$ is introduced (i.e. 0.2 mL of $NH_3$) both the yields of carbon products and methyl carbamate increase considerably. Indeed, the lack of available $NH_3$ reduces the reactivity of such intermediate decreasing the production and subsequent desorption of urea in favour of the desorption of the methyl carbamate, which shows a smaller kinetic constant. Even though the production of urea increases with $NH_3$ concentration, the total carbon yields are higher for 2 mL of $NH_3$ than for 1 mL. Such behaviour has been attributed to the increment in the total reaction pressure as the different $NH_3$ concentration results in different solid-liquid phase equilibrium at 120 °C. Accordingly, the final reaction pressures were 3 bars, 6 bars and 8 bars, respectively. Accordingly, the urea selectivity (derived only from the supernatant) increases from 28 % (0.2 mL of $NH_3$) to 95 % (1 mL of $NH_3$) and 97 % (2 mL of $NH_3$).

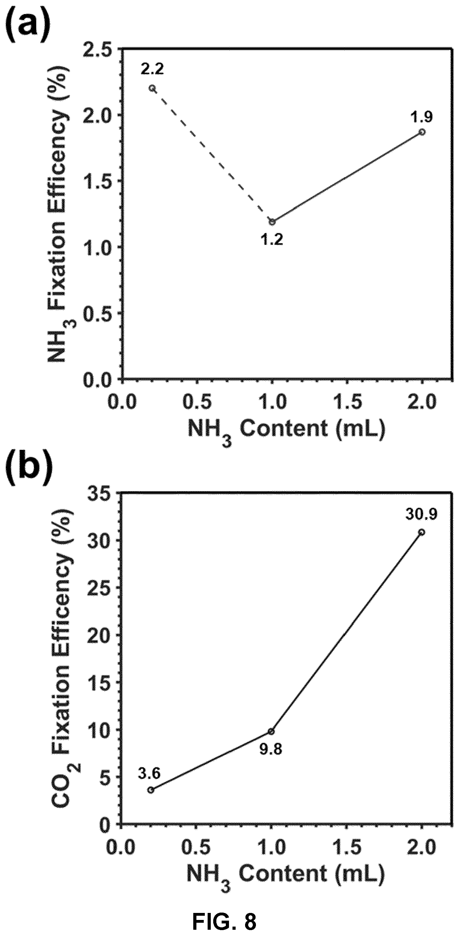

On the other hand, the urea efficiency with respect the initial $NH_3$ and $CO_2$ content is plotted in Figures 8a and 8b, respectively. Surprisingly, none of the curves show asymptotic stability as when doubling the $NH_3$ concentration the increment in the efficiency in Figure 8a almost doubles, while the efficiency towards $CO_2$ fixation (Figure 8b) multiplies by a factor >3. Note that the high efficiency obtained at 0.2 mL in Figure 8a has been attributed to the high $NH_3$ absorption capacity of upp-HAp, even at very low concentrations, and thus, cannot be considered as part of the kinetics of the reaction when considering the industrial viability of the system. Accordingly, similar behaviours have been reported for upp-HAp under very low $CO_2$ concentrations. Such results confirm the great efficiency of upp-HAp as a catalyst and further suggest that higher efficiencies could be achieved by increasing the $NH_3$ concentration of the reaction. Unfortunately, this assumption has not been experimentally corroborated as the reactor used cannot properly operate at such strong basic media. Nonetheless and despite of the contribution of the concentration of $NH_3$ to the total reaction pressure, the $CO_2$ fixation efficiency reported is extraordinarily high considering the reaction conditions. In this sense, the reaction kinetics of urea appear to be faster than the carbon-based products obtained from only $CO_2$ fixation reactions. More specifically, the fast desorption of urea seems to compensate the energetic barriers of the C-N bond generation in front of the C-C and/or C-O bond formation. Consequently, apart from the generation of a highly valuable chemical product such as urea, incorporating green $NH_3$ in a reaction catalysed by upp-HAp can be extremely advantageous as a $CO_2$-revalorisation strategy.

*2.5 Towards sustainable Bazarov reactions: comparison and limits of upp-HAp*

Considering the $NH_3$ efficiency towards urea, the advantages of using the upp-HAp catalyst with respect the industrial Bazarov reaction clearly arise. Certainly, the maximum efficiency obtained experimentally (~2 %, see Figure 8a) competes directly with the industrial efficiencies reported (< 10 %) but with the remarkable great advantage that the reaction is successfully achieved at almost half of the temperature and at 300 times less pressure. Moreover, and considering other metallic complexes, upp-HAp is a green and highly abundant catalyst (calcium is the 5th most abundant metal in earth's crust, 4.1% while phosphorus is the 12th most abundant in earth's crust). Thus, using upp-HAp not only represent a great sustainable approach for the Bazarov reaction, but also a great economical solution for the existent industrial technology as the infrastructure and energetic costs associated with the mild reaction conditions that definitely compensates the almost negligible economic costs of using upp-HAp as a catalyst.

At this point, the energetic cost of the synthesis of urea from $N_2$ and $CO_2$ (single-step) catalysed by upp-HAp, as compared with the catalytic reactions presented above need to be re-visited. To do so, several reactions with $N_2$ and $CO_2$ were carried out in the same reactor. However, in the range of 1 to 6 bars of pressure (50:50) the yield of urea was close to 0 (not shown). This result is in agreement with the literature which, among other strategies like the use of NO gas, reports the activation of $N_2$ assisted by UV irradiation.

Accordingly, a 120 °C, 6 bar of $CO_2$ and $N_2$ reaction exposed to UV irradiation for 24 °C was conducted. However, despite pressure values being six times higher and the reaction being carried out in the presence of UV irradiation (which increases the energetic demands of the reaction), the yield of urea obtained in the comparative reaction was $0.75 \pm 0.05$ mmol/$g_{cat}$ with a selectivity of 80 %. Such result is below the maximum yield reported for the $NH_3$ and $CO_2$ reactions (~1 mmol/$g_{cat}$ and selectivity of 97 %) at 120 °C and 1 bar of pressure of $CO_2$. Therefore, the results presented suggest that a double-step reaction for the sustainable synthesis of urea could be greener and more efficient than the single-step approach. That is because upp-HAp can efficiently catalyse the Bazarov reaction at competitive industrial yields but under very mild conditions that can be reached using renewable sources. Consequently, the challenge can be specifically reduced to develop optimised processes to obtain green $NH_3$, without the need to design multifunctional catalysts capable of promoting C-N bonds.

Finally, the limits of the upp-HAp for the $NH_3$ and $CO_2$ reaction were explored. While a reaction at 95 °C (24 h, 1 mL of $NH_3$) produced $0.25 \pm 0.03$ mmol/$g_{cat}$ of urea, the temperature limit was found at 70 °C (6 h reaction and 1 mL of $NH_3$, no urea is detected). Nonetheless, similar levels of methyl carbamate were measured (6.67 $\mu$mol/$g_{cat}$ and 8.23 $\mu$mol/$g_{cat}$, at 95 °C and 70 °C; respectively). All the yields obtained for both reactions are listed in Table 3. Locating the temperature limit below 100 °C represents a great advantage both industrially and from a sustainable point of view.

**Table 3.** Yields (in $\mu$mol/g$_{cat}$) of the reactions conducted at 70 °C and 95 °C.

| | Ethanol | Acetic Acid | Acetone | Formic Acid | Methyl Carbamate | Urea |
|---|---|---|---|---|---|---|
| **70 °C** | | | | | | |
| Catalyst | 0.44 | 0.39 | 0 | 2.53 | 0.61 | 0 |
| Supernatant | 13.25 | 5.81 | 0 | 40.05 | 8.23 | 0 |
| **120 °C** | | | | | | |
| Catalyst | 0.59 | 0.52 | 0.08 | 3.72 | 0.67 | 0 |
| Supernatant | 20.05 | 32.36 | 3.87 | 101.59 | 6.67 | 246 |

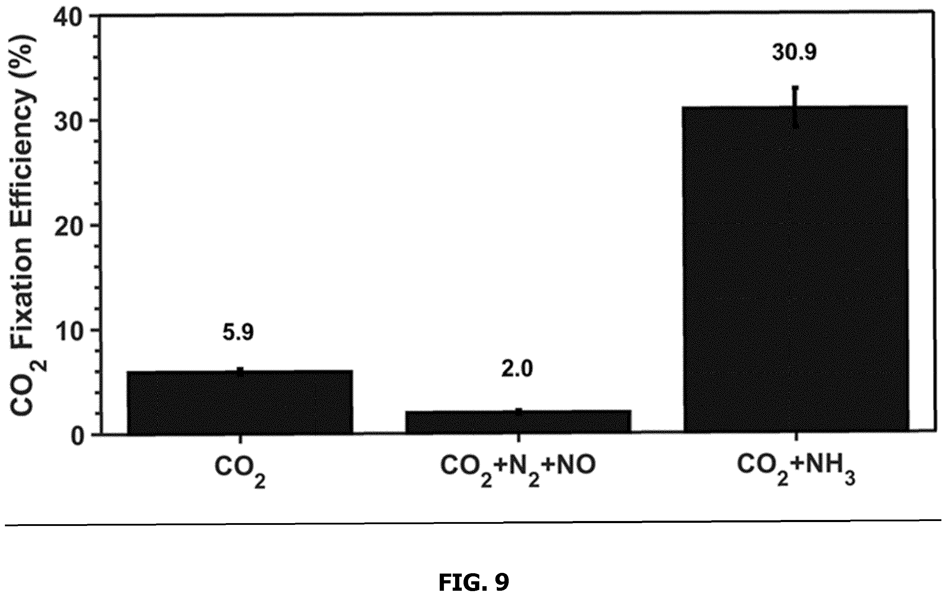

Finally, Figure 9 compares $CO_2$ fixation efficiencies obtained for different atmospheres: pure $CO_2$ (left), $CO_2+N_2$ +NO (314 ppm) (centre) and $CO_2+NH_3$ (liquid, 2 mL pure $NH_3$) (right). Other than the specific reagents, all the remaining conditions were identical in the three experiments (120 °C, total pressure 1 bar, 44.8 mL of $H_2O$ and 24 hours; using the upp-HAp catalyst). As it can be seen, there is a clear increase in the $CO_2$ fixation efficiency in the $CO_2+NH_3$ reactions assisted by these permanently polarized catalysts.

## Example 3 - Extension of the TSP treatment to other materials: a proof-of concept

### 3.1 TSP treatment on $SiO_2$

In order to extend and demonstrate the feasibility of the catalytic activation through the permanent polarisation treatment on other materials, permanently polarised SiOz was prepared and tested for identical reaction conditions. In this sense, SiOz was chosen as a representative candidate because it does not show any specific catalytic activity a priory, but it can present a characteristic piezoelectricity response, often related to the polarisation approach. Nonetheless, it is important highlighting the fact that the TSP treatment is capable to induce a permanently polarised state. To avoid any crystallographic phase transition, the TSP treatment was carried out at 500 °C. Thus, SiOz nanopowder (5-20 nm, Sigma-Aldrich) were subjected to the TSP treatment. To do so, the SiOz nanopowder was compressed into pellets. The experimental TSP conditions used were 500 °C and 1000 V for 1 h.

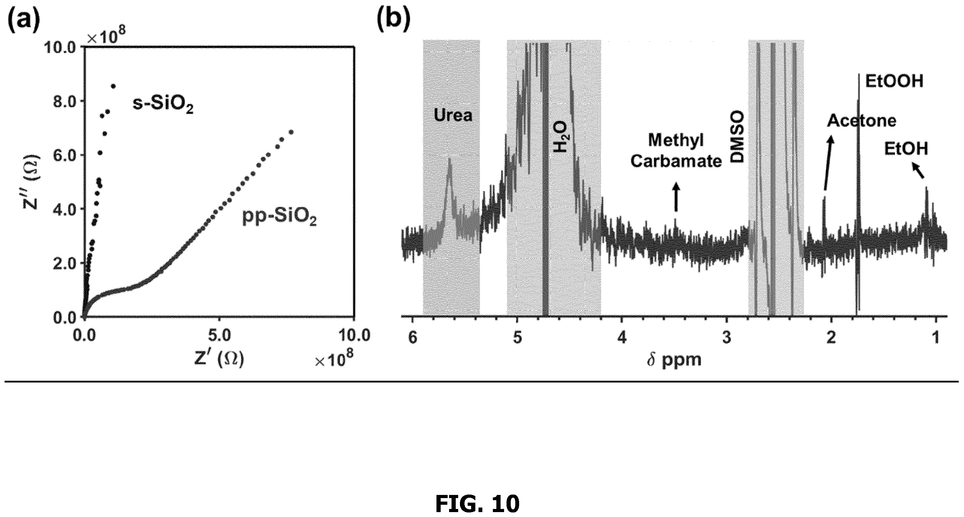

The electrical properties of permanently polarised SiOz (pp-$SiO_2$) were compared with a SiOz sample exposed to 500 °C but without applying the electrical field (s-$SiO_2$). As it can be observed in the bode plot in Figure 10a, the electrical properties of both samples measured by means of the EIS technique differ significantly. While for s-$SiO_2$ a capacitive behaviour is detected, pp-$SiO_2$ shows clear enhanced electrical transport properties evidenced by the semi-circle response, characteristic from a semi-conductor. Hence, the permanent effect of the TSP treatment on pp-$SiO_2$ is demonstrated.

Accordingly, the $^1$H NMR spectra of the supernatant in Figure 10b collected after the reaction using pp-$SiO_2$ as catalyst (120 °C, 1 bar $CO_2$ and 1 mL $NH_3$ for 24 hours) reveals the successful synthesis of urea. The presence of methyl carbamate is also detected, being in agreement with the results reported for upp-HAp. The yields of urea and the other products are summarised in Table 4.

**Table 4.** Yields (in $\mu$mol/g$_{cat}$) of the reactions conducted at 120 °C, 1 bar $CO_2$ 1 mL of $NH_3$ and 24 h, using pp-$SiO_2$ as a catalyst.

| pp-$SiO_2$ | EtOH | EtOOH | Acetone | HCOOH | $\Sigma$Carbon Products | Methyl Carbamate | Urea |
|---|---|---|---|---|---|---|---|
| Super na-tant | 23.45 $\pm$2.0 | 38.44 $\pm$3.71 | 7.57 $\pm$1.06 | 82.40 $\pm$13.16 | 151.86 | 13.68 $\pm$0.69 | 637.26 $\pm$41.41 |

Even though the urea yield obtained is higher, the selectivity towards urea decreases considerably (< 80 %). Overall, the versatility and suitability of the TSP treatment for the catalytic activation of different materials, resulting in the advantageous revalorisation of $CO_2$ through the production urea from $CO_2$ and $NH_3$ under mild conditions is demonstrated.

## Example 4 - Conclusions

The production of urea from $NH_3$ and $CO_2$ catalysed by upp-HAp under mild conditions (i.e. 120 °C and 1 bar of

$CO_2$ pressure; without applying any electrical currents or UV irradiation) has been demonstrated. As studied by Raman microscopy and XPS techniques upp-HAp shows high stability and great $NH_3$ absorption on its acid sites. Moreover, the surface charge accumulation of upp-HAp (main responsible of the catalytic activity of upp-HAp) is shown to be optimal under the basic media conditions of the reaction. Accordingly, both the selectivity and the urea efficiency with respect the initial $NH_3$ concentration can reach 97 % and ~2 %, respectively. The efficiency reported is in the same range of the current industrial Bazarov reactions. However, the reaction conditions achieved, and the nature of the catalyst suppose a great advantage for transitioning towards the complete sustainable production of urea by means of green $NH_3$ and $CO_2$. Because of upp-HAp, the $N_2$ activation to produce $NH_3$ can be assessed independently resulting in a more favourable energetic balance. Finally, the fast desorption of urea from upp-HAp (as compared with other carbon-based products) opens the door to a highly efficient strategy for $CO_2$-revalorisation as shown by the ~30 % of efficiency with respect to $CO_2$ fixation.

**Claims**

1. A method for the synthesis of urea from carbon dioxide ($CO_2$) and ammonia ($NH_3$), wherein the method comprises contacting a gaseous stream of $CO_2$ with an aqueous solution of $NH_3$ in the presence of a permanently polarised catalyst at a temperature of 70-220°C and at a total gas pressure of 0.01-250 bar.

2. The method according to claim 1, wherein the permanently polarised catalyst is a permanently polarised polymeric catalyst or a permanently polarised ceramic catalyst.

3. The method according to claim 2, wherein the permanently polarised polymeric catalyst is selected from the group consisting of polyphosphines, metal-functionalised conjugated microporous polymers, metalloporphyrin-based porous ionic polymers, polylactic acid (PLA), polyethylene terepthalate (PET), polyethylene terephthalate glycol (PETG), thermoplastic polyurethane (TPU), poly(3,4-ethylenedioxythiophene) (PEDOT), polyaniline (PANI), polypyrrole (PPy), polystyrene (PS), and combinations thereof.

4. The method according to claim 2, wherein the ceramic material at the basis of the permanently polarised ceramic catalyst is selected from the group consisting of hydroxyapatite ($Ca_5(PO_4)_3(OH)$), alumina ($Al_2O_3$), zirconia ($ZrO_2$), titania ($TiO_2$), vanadium(II) oxide (vanadium monoxide, VO), vanadium(III) oxide (vanadium sesquioxide or trioxide, $V_2O_3$), vanadium(IV) oxide (vanadium dioxide, $VO_2$), vanadium(V) oxide (vanadium pentoxide, $V_2O_5$), ferrous (Fe(II)) oxide, ferric (Fe(III)) oxide, silica ($SiO_2$), zeolite (with general formula $M^{n+}_{1/n}(AlO_2)^-(SiO_2)_x \cdot yH_2O$ where $M^{n+}_{1/n}$ is either a metal ion or hydrogen), montmorillonite (($Na,Ca)_{0.3}(Al,Mg)_2Si_4O_{10}(OH)_2 \cdot nH_2O$), $PbTiO_3$, $Pb/Mg_{0.3}Nb_{0.6})O_3$), $BaTiO_3$, Perovskite and combinations thereof.

5. The method according to claim 4, wherein the permanently polarised ceramic catalyst is permanently polarised hydroxyapatite, permanently polarised silicon dioxide, permanently polarised zirconia, permanently polarised vanadium(II) oxide (vanadium monoxide, VO), permanently polarised vanadium(III) oxide (vanadium sesquioxide or trioxide, $V_2O_3$), permanently polarised vanadium(IV) oxide (vanadium dioxide, $VO_2$), or permanently polarised vanadium(V) oxide (vanadium pentoxide, $V_2O_5$).

6. The method according to any one of claims 1 to 5, wherein the catalytic materials are decorated with metallic catalytic particles, preferably wherein the metallic catalytic particles are selected from the group consisting of Au, Fe, Cu, Mo, Pt, Pd, and combinations thereof.

7. The method according to any one of claims 1 to 6, wherein the permanently polarised catalyst is a non-porous material or a porous material.

8. The method according to any one of claims 1 to 7, wherein the $CO_2$ pressure is between 0.01-250 bar, preferably 0.1-20, more preferably 1-6 bar.

9. The method according to any one of claims 1 to 8, wherein the $NH_3$ concentration is $0.5 \times 10^{-3}$M - 100.0 M, preferably $1.0 \times 10^{-3}$M - 20.0 M, more preferably $1.0 \times 10^{-2}$M-5.0 M.

10. The method according to any one of claims 1 to 9, wherein the volumetric ratio of aqueous solution to catalyst is between 10000:1 and 0.1:1, preferably between 1000:1 and 1:1, more preferably between 1000:1 to 100:1.

11. The method according to any one of claims 1 to 10, wherein the volumetric ratio of gaseous $CO_2$ to aqueous solution is between 100:1 and 1:100, preferably between 10:1 and 1:100, more preferably between 5:1 to 1:10.

12. The method according to any one of claims 1 to 11, wherein the temperature is between 70 and 220°C, preferably between 80 and 150°C, more preferably between 90 and 140°C.

13. The method according to any one of claims 1 to 12, wherein the total gas pressure is between 0.2 and 250 bar, preferably between 0.8 and 10 bar, more preferably between 1 and 6 bar.

14. The method according to any one of claims 1 to 13, wherein the reaction time is between 10 minutes to 48 hours, preferably between 6 hours to 42 hours, more preferably between 18 hours to 36 hours.

15. Use of a permanently polarised catalyst for:

   - reducing $CO_2$ concentration present in a gaseous stream;
   - reducing $NH_3$ concentration present in an aqueous solution; and/or
   - producing urea,

by implementing a method according to any one of claims 1 to 14.

**FIG. 1**

**FIG. 2**

1 Gas input
2 Gas output
3 UV Lamp
4 Heater
5 Teflon holder for the catalyst
6 Liquid ($H_2O$+$NH_3$)

FIG. 3

FIG. 4

FIG. 5

**FIG. 6**

**FIG. 7**

EP 4 686 719 A1

FIG. 8

**FIG. 9**

EP 4 686 719 A1

FIG. 10

Europäisches
Patentamt

European
Patent Office

Office européen
des brevets

## EUROPEAN SEARCH REPORT

**Application Number**

**EP 24 38 2863**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 3 932 504 A (CHEN LARRY P ET AL) 13 January 1976 (1976-01-13) * See claim 1; examples * | 1-15 | INV. C07C273/04 B01J35/23 |
| A,D | WO 2018/024727 A1 (B BRAUN SURGICAL SA [ES]; UNIV CATALUNYA POLITECNICA [ES]) 8 February 2018 (2018-02-08) * See claim 12; pages 35-41 * | 1-15 | |
| A,D | WO 2023/139032 A1 (UNIV CATALUNYA POLITECNICA [ES]; B BRAUN SURGICAL SA [ES]) 27 July 2023 (2023-07-27) * See claims; page 19 * | 1-15 | |

**TECHNICAL FIELDS SEARCHED (IPC)**

C07C
B01J

The present search report has been drawn up for all claims

| Place of search | Date of completion of the search | Examiner |
|---|---|---|
| The Hague | 20 January 2025 | Menchaca, Roberto |

EPO FORM 1503 03.82 (P04C01)

## ANNEX TO THE EUROPEAN SEARCH REPORT
## ON EUROPEAN PATENT APPLICATION NO.

EP 24 38 2863

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2025

| Patent document cited in search report | | Publication date | Patent family member(s) | | Publication date |
|---|---|---|---|---|---|
| US 3932504 | A | 13-01-1976 | AU | 500371 B2 | 17-05-1979 |
| | | | CA | 1049556 A | 27-02-1979 |
| | | | US | 3932504 A | 13-01-1976 |
| WO 2018024727 | A1 | 08-02-2018 | AU | 2017308004 A1 | 07-02-2019 |
| | | | BR | 112019002072 A2 | 14-05-2019 |
| | | | CA | 3031604 A1 | 08-02-2018 |
| | | | CN | 109803919 A | 24-05-2019 |
| | | | EP | 3494087 A1 | 12-06-2019 |
| | | | IL | 264348 A | 28-02-2019 |
| | | | JP | 7023955 B2 | 22-02-2022 |
| | | | JP | 2019534236 A | 28-11-2019 |
| | | | KR | 20190039172 A | 10-04-2019 |
| | | | MY | 194363 A | 29-11-2022 |
| | | | SA | 519401013 B1 | 15-02-2023 |
| | | | SG | 11201900618W A | 27-02-2019 |
| | | | US | 2020180960 A1 | 11-06-2020 |
| | | | WO | 2018024727 A1 | 08-02-2018 |
| WO 2023139032 | A1 | 27-07-2023 | CN | 118613324 A | 06-09-2024 |
| | | | EP | 4215271 A1 | 26-07-2023 |
| | | | WO | 2023139032 A1 | 27-07-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018024727 A1

- WO 2023139032 A1

**Non-patent literature cited in the description**

- **JORDI SANS et al.** Synthesis of urea from CO2 and N2 fixation under mild conditions using polarised hydroxyapatite as a catalyst. *Sustainable Energy Fuels*, 2024, vol. 8, 1473

- **J. SANS et al.** Hydroxyapatite-based biphasic catalysts with plasticity properties and its potential in carbon dioxide fixation. *Chem. Eng. J.*, 2022, vol. 433, 133512